# EUROPEAN PATENT APPLICATION

(11) **EP 1 577 310 A1**
(43) Date of publication of application: **21.09.2005**
(21) Application number: 03778791.8
(22) Date of filing: 10.12.2003
(51) Int. Cl.: C07D 407/06, C07D 317/60, C07D 305/12

(54) **PROCESSES FOR PRODUCING OPTICALLY ACTIVE 2-THIOMETHYL-3-PHENYLPROPIONIC ACID DERIVATIVE AND FOR PRODUCING INTERMEDIATE THEREFOR**

(30) Priority: 27.12.2002 JP 2002378842
(71) Applicant: KANEKA CORPORATION, Osaka-shi, Osaka 530-8288 (JP)
(72) Inventor: OHISHI, Takahiro, c/o KANEKA CORPORATION, Takasago-shi, Hyogo 676-0027 (JP); MORI, Kohei, c/o KANEKA CORPORATION, Takasago-shi, Hyogo 676-0027 (JP); KINOSHITA, Koichi, c/o KANEKA CORPORATION, Takasago-shi, Hyogo 676-0027 (JP); MAEHARA, Katsuji, c/o KANEKA CORPORATION, Takasago-shi, Hyogo 676-0027 (JP); KAWASAKI, Hiroaki, c/o KANEKA CORPORATION, Takasago-shi, Hyogo 676-0027 (JP); NAGASHIMA, Nobuo, c/o KANEKA CORPORATION, Takasago-shi, Hyogo 676-0027 (JP); FUSE, Yoshihide, c/o KANEKA CORPORATION, Takasago-shi, Hyogo 676-0027 (JP)
(74) Representative: Vossius & Partner
(86) International application number: PCT/JP2003/015821
(87) International publication number: WO 2004/060885

(57) **Abstract**

The present invention provides a process for simply producing an optically active 2-thiomethyl-3-phenylpropionic acid derivative useful as an intermediate for medicines from inexpensive raw materials. An optically active 2-hydroxymethyl-3-phenylpropionic acid ester derivative which can be relatively easily obtained by asymmetric reduction reaction with an enzyme is cyclized to an optically active β-lactone derivative which is then reacted with a sulfur compound to produce an optically active 2-thiomethyl-3-phenylpropionic acid derivative in high yield.

## Description

### Technical Field

The present invention relates to a process for producing an optically active 2-thiomethyl-3-phenylpropionic acid derivative useful as an intermediate for medicines and the like, an intermediate useful for synthesis thereof, and a process for producing the intermediate.

### Background Art

Known processes for producing optically active 2-thiomethyl-3-phenylpropionic acid derivatives are roughly divided into the three processes including a process of resolving a racemic 2-thiomethyl-3-phenylpropionic acid derivative, a method of inducing a target compound from a non-optically active material, and a method of inducing a target compound using an optically active compound as a raw material.

An example of the process of resolving a racemic derivative is a process of converting a racemic 2-thiomethyl-3-phenylpropionic acid derivative to a salt with an optically active amine or bonding the derivative with an optically active amino acid to form a diastereomer mixture, and then resolving the mixture by crystallization. Examples of the amine salt include an ephedrine salt (Japanese Unexamined Patent Application Publication No. 08-59606), an N-isopropyl alaninol salt (J. Med. Chem., 1992, 35(3), pp. 602-608), and a 1-amino-indanol salt (Japanese Unexamined Patent Application Publication No. 11-228532). Examples of the amino acid bonded include alanine (PCT Japanese Translation Patent Publication No. 4-501868). Another process has been also reported, in which an acetyl group on a sulfur atom is stereoselectively hydrolyzed with an enzyme (US 5177006A and Biotechnol. Appl. Biochem., 1992, 16(1), pp. 34-37). However, these processes have a maximum yield of 50% due to resolution, and are far from being practical and economical for industrial production.

A known example of the process of inducing from a non-optically active raw material uses asymmetric hydrogenation reaction of α-hydroxymethylcinnamic acid derivative or its ester. There have been reports of examples using several asymmetric catalysts (FR2772027A1, Japanese Unexamined Patent Application Publication No. 2000-229907, Aust. J. Chem., 1998, 51(6), pp. 511-514, Enantiomer, 1998, 3(2), pp. 191-195, etc.). However, any one of these examples is disadvantageous in that the catalyst is expensive and difficult to stably obtain or has low selectivity, and is thus impractical.

As the process of inducing from an optically active raw material, there have been reports of a process of reacting optically active 2-hydroxymethyl-3-phenylpropionic acid with a Mitsunobu reagent and potassium tioacetate (PCT Japanese Translation Patent Publication No. 11-503470) and a process of converting an optically active 2-sulfonyloxy-3-phenyl-1-propanol derivative or an optically active 2-hydroxymethyl-3-phenylpropionic acid derivative to an optically active 2-sulfonyloxy-3-phenylpropionic acid derivative and then reacting the product with a sulfur compound to form a target compound (WO98/05634). However, these processes are disadvantageous in that the target compound cannot be easily isolated and purified, and the efficiency of reaction with the sulfur compound is insufficient, and thus have needs to be improved for industrial production.

### Disclosure of Invention

In consideration of the above-described situation, an object of the present invention is to provide a practical process capable of simply and industrially advantageously producing an optically active 2-thiomethyl-3-phenylpropionic acid derivative.

As a result of intensive research in consideration of above-described situation, the present inventors found a process for producing an optically active 2-thiomethyl-3-phenylpropionic acid derivative by converting an optically active 2-sulfonyloxymethyl-3-phenylpropionic acid derivative or an optically active 2-hydroxymethyl-3-phenylpropionic acid derivative to an optically active β-lactone derivative, and then reacting the resulting derivative with a sulfur compound. This finding resulted in completion of the present invention.

Namely, the present invention relates to a process for producing an optically active β-lactone derivative represented by formula (2): 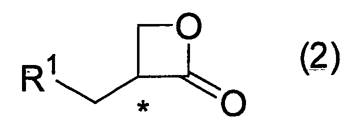 (wherein * represents an asymmetric carbon atom, and R¹ represents a phenyl group which may be substituted), the process comprising cyclizing an optically active 2-sulfonyloxymethyl-3-phenylpropionic acid derivative represented by formula (1) : 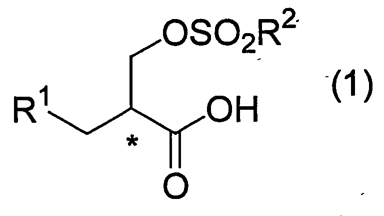 (wherein * and R¹ represent the same as the above, and R² represents a C₁-C₁₀ alkyl group which may be substituted or a C₆-C₂₀ aryl group which may be substituted).

Also, the present invention relates to a process for producing an optically active 2-thiomethyl-3-phenylpropionic acid derivative represented by formula (7) : 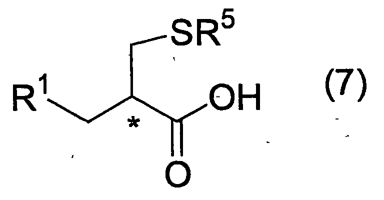 (wherein * and R¹ represent the same as the above, and R⁵ represents a C₁-C₁₀ alkyl group which may be substituted, a C₆-C₂₀ aryl group which may be substituted, a C₂-C₂₀ acyl group which may be substituted, or a C₇-C₂₀ aroyl group which may be substituted), the process comprising reacting an optically active β-lactone derivative represented by formula (2) with a sulfur compound represented by formula (6):

R⁴SR⁵ (6)

(wherein R⁴ represents a hydrogen atom or an alkali metal atom, and R⁵ represents the same as the above).

The present invention further relates to a process for producing an optically active 2-thiomethyl-3-phenylpropionic acid derivative represented by formula (7), the process comprising cyclizing an optically active 2-hydroxymethyl-3-(3,4-methylenedioxyphenyl)propionic acid derivative represented by formula (8): 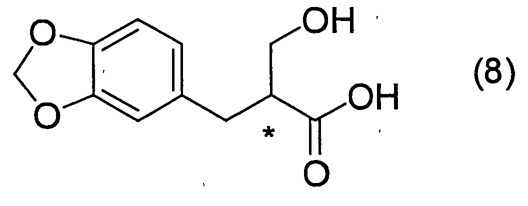 (wherein * represents the same as the above) to form an optically active β-lactone derivative represented by formula (2), and then reacting the β-lactone derivative represented by formula (2) with a sulfur compound represented by formula (6)

The present invention further relates to an optically active β-lactone derivative represented by formula (10): 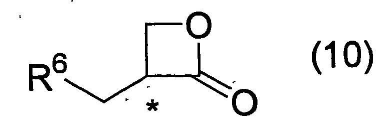 (wherein * represents the same as the above, and R⁶ represents a substituted phenyl group).

The present invention further relates to an optically active 2-sulfonyloxymethyl-3-phenylpropionic acid ester derivative represented by formula (11): 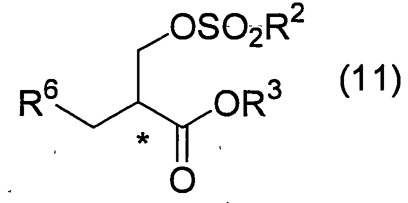 (wherein *, R², R³, and R⁶ represent the same as the above).

The present invention further relates to an optically active 2-hydroxymethyl-3-phenylpropionic acid derivative represented by formula (12): 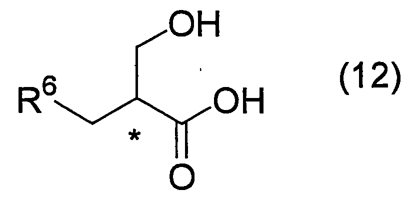 (wherein * and R⁶ represent the same as the above).

The present invention will be described in detail below. In the specification, the term "optically active" means that in a compound having one asymmetric carbon atom, one of two enantiomers having asymmetric carbon atoms with different absolute configurations exists at a higher ratio.

First, raw materials used in the present invention, and production processes therefor will be described.

The raw materials used in the present invention include an optically active 2-sulfonyloxymethyl-3-phenylpropionic acid derivative represented by formula (1): 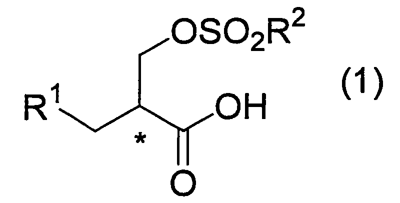 and an optically active 2-hydroxymethyl-3-(3,4-methylenedioxyphenyl)propionic acid derivative represented by formula (8):

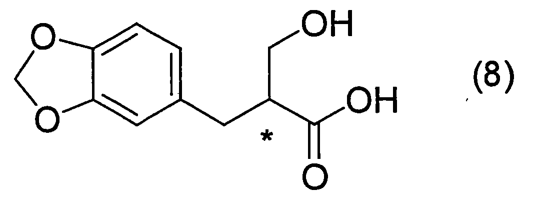

In formulae (1) and (8), * represents an asymmetric carbon atom. In formula (1), R¹ represents a phenyl group which may be substituted. Examples of a phenyl group which may be substituted include phenyl, 2, 3-methylenedioxyphenyl, 2,3-ethylenedioxyphenyl, 2,3-propylenedioxyphenyl, 3,4-methylenedioxyphenyl, 3,4-ethylenedioxyphenyl, 3,4-propylenedioxyphenyl, o-tolyl-, m-tolyl, p-tolyl, 2,3-xylyl, 2,4-xylyl, 2,5-xylyl, 2,6-xylyl, 3,4-xylyl, o-phenoxyphenyl, m-phenoxyphenyl, p-phenoxyphenyl, o-phenylphenyl, m-phenylphenyl, p-phenylphenyl, o-chlorophenyl, m-chlorophenyl, p-chlorophenyl, o-bromophenyl, m-bromophenyl, p-bromophenyl, o-fluorophenyl, m-fluorophenyl, p-fluorophenyl, o-nitrophenyl, m-nitrophenyl, p-nitrophenyl, o-cyanophenyl, m-cyanophenyl, p-cyanophenyl, o-hydroxyphenyl, m-hydroxyphenyl, p-hydroxyphenyl, o-methoxyphenyl, m-methoxyphenyl, p-methoxyphenyl, 2,3-dimethoxyphenyl, 2,4-dimethoxyphenyl, 2,5-dimethoxyphenyl, 2,6-dimethoxyphenyl, 3,4-dimethoxyphenyl, 2,3-difluorophenyl, 2,4-difluorophenyl, 2,5-difluorophenyl, 2,6-difluorophenyl, 3,4-difluorophenyl, 2,3-dihydroxyphenyl, 2,4-dihydroxyphenyl, 2,5-dihydroxyphenyl, 2,6-dihydroxyphenyl, and 3,4-dihydroxyphenyl. Among these groups, phenyl and 3,4-methylenedioxyphenyl are preferred.

In formula (1), R² represents a C₁-C₁₀ alkyl group which may be substituted, or a C₆-C₂₀ aryl group which may be substituted. The number of carbon atoms is a value excluding that of a substituent. This is true for the description below unless otherwise specified.

Examples of the C₁-C₁₀ alkyl group which may be substituted include methyl, ethyl, propyl, isopropyl, butyl, isobutyl, sec-butyl, tert-butyl, trifluoromethyl, and benzyl. Examples of the C₆-C₂₀ aryl group which may be substituted include phenyl, p-tolyl, o-tolyl, m-tolyl, 2,3-xylyl, 2,4-xylyl, 2,5-xylyl, 2,6-xylyl, 3,4-xylyl, o-chlorophenyl, m-chlorophenyl, p-chlorophenyl, o-nitrophenyl, m-nitrophenyl, and p-nitrophenyl. Among these groups, methyl, p-tolyl, phenyl, benzyl, and trifluoromethyl are preferred for obtaining a target compound with high purity and low production of impurities.

The present inventors found that the optically active 2-hydroxymethyl-3-(3,4-methylenedioxyphenyl)propionic acid derivative represented by formula (8) is a novel compound having usefulness for synthesis of an optically active 2-thiomethyl-3-phenylpropionic acid derivative.

The optically active 2-sulfonyloxymethyl-3-phenylpropionic acid derivative represented by formula (1) can be obtained by, for example, asymmetrically reducing a corresponding racemic 2-formyl-3-phenylpropionic acid ester derivative with an enzyme to form an optically active 2-hydroxymethyl-3-phenylpropionic acid ester derivative represented by formula (3) (Japanese Unexamined Patent Application Publication No. 60-199383), sulfonylating the optically active 2-hydroxymethyl-3-phenylpropionic acid ester derivative (3), and then hydrolyzing it. 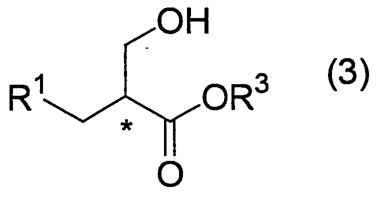 The optically active 2-hydroxymethyl-3-(3,4-methylenedioxyphenyl)propionic acid derivative represented by formula (8) can be obtained by hydrolyzing a compound containing 3,4-methylenedioxyphenyl as R¹ of the optically active 2-hydroxymethyl-3-phenylpropionic acid ester derivative (3).

A process for producing the optically active 2-sulfonyloxymethyl-3-phenylpropionic acid derivative (1) from the optically active 2-hydroxymethyl-3-phenylpropionic acid ester derivative (3) will be described below.

In the optically active 2-hydroxymethyl-3-phenylpropionic acid ester derivative represented by formula (3), * and R¹ represent the same as the above, and R³ represents a C₁-C₁₀ alkyl group which may be substituted, or a C₆-C₂₀ aryl group which may be substituted.

Examples of the C₁-C₁₀ alkyl group which may be substituted include methyl, ethyl, propyl, isopropyl, butyl, isobutyl, sec-butyl, tert-butyl, trifluoromethyl, and benzyl. Example of the C₆-C₂₀ aryl group which may be substituted include phenyl, p-tolyl, o-tolyl, m-tolyl, 2,3-xylyl, 2,4-xylyl, 2,5-xylyl, 2,6-xylyl, 3,4-xylyl, o-chlorophenyl, m-chlorophenyl, p-chlorophenyl, o-nitrophenyl, m-nitrophenyl, and p-nitrophenyl. Among these groups, any one of methyl, ethyl, and tert-butyl is preferred for efficiently promoting the subsequent hydrolysis to obtain the target compound in high yield.

The optically active 2-hydroxymethyl-3-phenylpropionic acid ester derivative (3) may be sulfonylated by a general process, for example, reaction with a sulfonic acid halide represented by formula (4) in the presence of an appropriate base:

R²SO₂X (4)

In formula (4), R² represents the same as the above, and X represents a halogen atom. Examples of the halogen atom include an iodine atom, a bromine atom, and a chlorine atom, and a chlorine atom is preferred. Although the base allowed to coexist is not particularly limited as long as it can capture the produced acid, for example, tertiary amines such as triethylamine, diisopropylethylamine, pyridine, dimethylaminopyridine, and lutidine are preferred.

The amount of each of the sulfonic acid halide (4) and base used is preferably 1 to 5 times the moles of the optically active 2-hydroxymethyl-3-phenylpropionic acid ester derivative (3) and more preferably 1 to 2 times the moles from an economical viewpoint.

The reaction is performed in a proper solvent. Preferred examples of the solvent include benzene, substituted benzenes such as toluene, chloroalkanes such as methylene chloride, ethers such as tetrahydrofuran and diethyl ether, and alkanes such as he'xane and pentane. Among these solvents, toluene is more preferred.

The reaction temperature is preferably -10 to 50°C and more preferably -5 to 30°C. The reaction may be stopped when the raw material disappears, but the reaction time is preferably about 1 to 10 hours.

The above-described operation can produce an optically active 2-sulfonyloxymethyl-3-phenylpropionic acid ester derivative represented by formula (5): 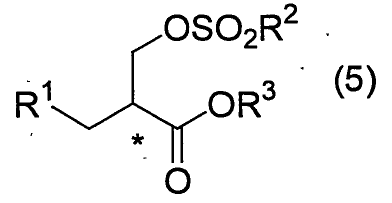 In formula (5), *, R¹, R², and R³ represent the same as the above.

The present inventor found a novel compound containing a group other than an unsubstituted phenyl group as R¹ in formula (5), i.e., an optically active 2-sulfonyloxymethyl-3-phenylpropionic acid ester derivative represented by formula (11): 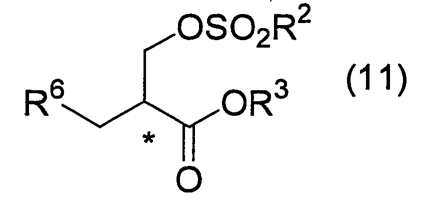 (wherein *, R², and R³ represent the same as the above, and R⁶ represents a phenyl group having a C₆-C₂₀ substituent), the compound having usefulness for synthesis of an optically active 2-thiomethyl-3-phenylpropionic acid derivative.

The optically active 2-sulfonyloxymethyl-3-phenylpropionic acid ester derivative (5) can be converted to the optically active 2-sulfonyloxymethyl-3-phenylpropionic acid derivative (1) by hydrolysis. The hydrolysis is preferably performed under acid conditions because a sulfonyloxy group is rapidly eliminated to form an unsaturated ester under alkaline conditions. However, when a substituent on a benzene ring may produce some reaction under the acid conditions, reaction conditions must be carefully determined. For example, the reaction conditions for a case in which a methylenedioxy group relatively weak against an acid is present on a benzene ring will be described in detail below.

As an acid, at least one acid is preferably, selected from the group consisting of acetic acid, formic acid, hydrochloric acid, sulfuric acid, p-toluenesulfonic acid, methanesulfonic acid, and trifluoromethanesulfonic acid, and combination of acetic acid and either sulfuric acid or p-toluenesulfonic acid is more preferred.

Either sulfuric acid or p-toluenesulfonic acid is preferably added as an aqueous solution. The concentration of the acid is preferably 2 to 30% by weight and more preferably 5 to 20% by weight. The amount of the acid added is preferably 0.1 to 5 times and more preferably 0.25 to 2 times by mole based on the optically active 2-sulfonyloxymethyl-3-phenylpropionic acid ester derivative (5). With respect to the mixing ratio to acetic acid, the amount of acetic acid used is preferably 2 to 20 times and more preferably 2 to 10 times by weight of an aqueous sulfuric acid solution or aqueous p-toluenesulfonic acid solution.

The reaction temperature is preferably in a range of 50°C to a reflux temperature, and more preferably a range from 60°C to 100°C. The reaction time is preferably about 3 to 48 hours and more preferably 3 to 30 hours because an excessively long reaction time brings about a decrease in yield due to decomposition of the product.

After the reaction, post-treatment can be performed by, for example, neutralizing the added sulfuric acid or p-toluenesulfonic acid with an alkali, distilling off acetic acid under reduced pressure, and then adding water and an organic solvent to the residue for extraction. As the alkali, for example, an alkali metal hydroxide, an alkali metal hydrogen carbonate, and an alkali metal carbonate are preferred. In particular, sodium hydroxide, potassium hydroxide, lithium hydroxide, sodium hydrogen carbonate, potassium hydrogen carbonate, potassium carbonate, and sodium carbonate are preferred. As the extraction solvent, benzene, toluene, xylene, methylene chloride, chloroform, carbon tetrachloride, diethyl ether, and ethyl acetate are preferred, and toluene and ethyl acetate are more preferred.

The crude product obtained by concentrating the resultant organic layer may be supplied to a next step without purification, or may be used after purification by, for example, chromatography or the like.

When the benzene ring is unsubstituted or substituted only by a group which may be not decomposed with an acid, the reaction can be performed by usual acid hydrolysis of esters. Examples of the acid used include, without limitation to, mineral acids such as hydrochloric acid and sulfuric acid, Lewis acids such as boron trichloride, trifluoroacetic acid, acetic acid, p-toluenesulfonic acid, methanesulfonic acid, and trifluoromethanesulfonic acid. Among these acids, hydrochloric acid, sulfuric acid, boron trichloride, trifluoroacetic acid, and p-toluenesulfonic acid are preferred.

As the reaction solvent, a mixed solvent containing an organic solvent and water, acetic acid, formic acid, or the like is used. Preferably, acetic acid, formic acid, or a mixed solvent containing water and an organic solvent miscible with water, such as dioxane, tetrahydrofuran, or an alcohol, is' used. The reaction temperature is not particularly limited as long as the reaction proceeds; but the temperature is preferably 0°C to a reflux temperature and more preferably about 0°C to 100°C. The reaction may be stopped when the yield of the target compound is maximized, and the reaction time is riot particularly limited. However, the reaction time is preferably about 1 to 48 hours.

Next, description will be made of a process for producing the optically active 2-hydroxymethyl-3-(3,4-methylenedioxyphenyl)propionic acid derivative (8) from a compound containing 3,4-methylenedioxyphenyl as R¹ of the optically active 2-hydroxymethyl-3-phenylpropionic acid ester derivative (3).

The optically active 2-hydroxymethyl-3-(3,4-methylenedioxyphenyl)propionic acid derivative (8) can be easily produced by hydrolysis of the compound containing 3,4-methylenedioxyphenyl as R¹ of the optically active 2-hydroxymethyl-3-phenylpropionic acid ester derivative (3). The hydrolysis process is not particularly limited, but the hydrolysis must be performed under alkaline conditions because the methylenedioxy group may be decomposed under acid conditions. Preferred examples of the alkali used include alkali metal hydroxides, alkali metal hydrogen carbonates, and alkali metal carbonates. In particular, sodium hydroxide, potassium hydroxide, lithium hydroxide, sodium hydrogen carbonate, potassium hydrogen carbonate, potassium carbonate, and sodium carbonate are preferred.

The reaction is performed in an aqueous solution or a mixed solvent containing water and an organic solvent miscible with water. As the organic solvent miscible with water, for example, methyl alcohol, ethyl alcohol, isopropyl alcohol, tetrahydrofuran, dioxane, and the like are preferred. The reaction temperature is preferably in a range of 0°C to a reflux temperature, and more preferably in a range of 0°C to 60°C because an excessively high temperature may cause racemization. The reaction is preferably stopped immediately after the disappearance of the raw material is confirmed. Since an excessively long time may cause racemization, the reaction is preferably performed for about 2 to 48 hours and then stopped.

When the hydrolysis is performed under acid conditions, the hydrolysis may be performed by a general process. Examples of the acid used include, without limitation to, mineral acids such as hydrochloric acid and sulfuric acid, Lewis acids such as boron trichloride, trifluoroacetic acid, acetic acid, p-toluenesulfonic acid, methanesulfonic acid, and trifluoromethanesulfonic acid. In particular, hydrochloric acid, sulfuric acid, boron trichloride, trifluoroacetic acid, and p-toluenesulfonic acid are preferred.

As the reaction solvent, acetic acid, formic acid, a mixed solvent containing an organic solvent and water, or the like is used. Preferably, acetic acid, formic acid, or a mixed solvent containing water and an organic solvent miscible with water, such as dioxane, tetrahydrofuran, or an alcohol, is used. The reaction temperature is not particularly limited as long as the reaction proceeds, but the temperature is preferably 0°C to a reflux temperature and more preferably about 0°C to 100°C. The reaction may be stopped when the yield of the target compound is maximized, and the reaction time is not particularly limited. However, the reaction time is preferably about 1 to 48 hours.

Next, description will be made of a process for producing the optically active β-lactone derivative represented by formula (2) of the present invention: 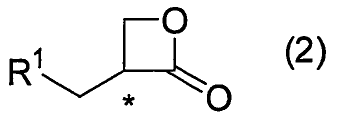 (wherein * and R¹ represents the same as the above). First, a process for cyclizing the optically active 2-sulfonyloxymethyl-3-phenylpropionic acid derivative (1) will be described.

The reaction in a mixed solvent containing water and a proper organic solvent can produce the generally unstable β-lactone derivative (2) in high yield because the optically active β-lactone derivative (2) produced by cyclization reaction is present in an organic layer, and thus hydrolytic ring-'opening reaction does not proceed. As the organic solvent used, at least one solvent is preferably selected from the group consisting of toluene, benzene, xylene, anisole, ethyl acetate, diethyl ether, methylene chloride, chloroform, and carbon tetrachloride. In particular, toluene and ethyl acetate are preferred.

The optically active 2-sulfonyloxymethyl-3-phenylpropionic acid derivative (1) is added to the organic solvent, and then water is added to the solution to start the reaction. In this reaction, the pH of the reaction mixture must be adjusted by adding an appropriate alkali. The alkali used is not particularly limited as long as the pH can be adjusted. Preferred examples of the alkali include alkali metal hydroxides, alkali metal hydrogen carbonates, and alkali metal carbonates. In particular, sodium hydroxide, potassium hydroxide, lithium hydroxide, sodium hydrogen carbonate, potassium hydrogen carbonate, potassium carbonate, and sodium carbonate are preferred. The pH is preferably adjusted to 4 or more with such an alkali, and particularly preferably adjusted in a range of 4 to 12 for obtaining the target compound in higher yield.

With respect to the mixing ratio between the water and the organic solvent, the volume of the organic solvent added is preferably 1 to 10 times and more preferably 1 to 5 times the volume of the water. The reaction temperature is preferably in a range of 0°C to a reflux temperature. An excessively low temperature may cause a low reaction rate, while an excessively high temperature may cause a reduction in yield due to decomposition of the product. Therefore, the reaction is more preferably performed in a range of 10°C to 50°C. The reaction may be stopped when the yield of the target compound is maximized, but the reaction time is preferably about 5 to 48 hours and particularly preferably about 5 to 30 hours.

After the reaction, the target compound, the optically active β-lactone derivative (2), is present in the organic layer and can thus be separated from the raw material only by a separation operation. The resultant organic layer can be supplied to a next step after concentration without purification, but it may be used after purification by, for example, chromatography or the like.

Next, description will be made of a process for producing the optically active β-lactone derivative (2) by cyclizing the optically active 2-hydroxymethyl-3-(3,4-methylenedioxyphenyl)propionic acid derivative represented by formula (8).

Synthesis of the optically active β-lactone derivative (2) by cyclization reaction of the optically active 2-hydroxymethyl-3-(3,4-methylenedioxyphenyl)propionic acid derivative (8) is not particularly limited as long as dehydration and condensation reaction can be performed. For example, cyclization can be performed by any one of the following five processes:
1) A process of cyclizing the optically active 2-hydroxymethyl-3-(3,4-methylenedioxyphenyl)propionic acid derivative represented by formula (8) in the presence of an azodicarboxylic acid ester represented by formula (13):

   R⁷O₂C-N=N-CO₂R⁸ (13)

   and a phosphine compound represented by formula (14):

   R⁹ ₃P (14)
2) A process of reacting with the sulfonic acid halide represented by formula (4) in the presence of a base.
3) A process using a condensing agent such as dicyclohexylcarbodiimide or the like.
4) A process using a mixed acid anhydride produced by reaction with a halogenated ester.
5) A process using an acid chloride produced by reaction with a chlorinating agent.

First, the cyclization process 1) will be described. In formula (13), R⁷ and R⁸ may be the same or different and independently represent a C₁-C₁₀ alkyl group which may be substituted or a C₁-C₂₀ aryl group which may be substituted.

Examples of the C₁-C₁₀ alkyl group which may be substituted include methyl, ethyl, propyl, isopropyl, butyl, isobutyl, sec-butyl, tert-butyl, trifluoromethyl, and benzyl. Examples of the C₆-C₂₀ aryl group which may be substituted include phenyl, o-tolyl, m-tolyl, p-tolyl, o-chlorophenyl, m-chlorophenyl, p-chlorophenyl, o-nitrophenyl, m-nitrophenyl, and p-nitrophenyl. In order to obtain the target compound in high yield, both R⁷ and R⁸ are preferably ethyl or isopropyl.

In formula (14), R⁹ represents a C₁-C₁₀ alkyl group which may be substituted, a C₁-C₁₀ alkoxy group which may be substituted, a C₆-C₂₀ aryloxy group which may be substituted, or a C₆-C₂₀ aryl group which may be substituted.

Examples of the C₁-C₁₀ alkyl group which may be substituted include methyl, ethyl, propyl, isopropyl, butyl, isobutyl, sec-butyl, tert-butyl, trifluoromethyl, and benzyl. Examples of the C₁-C₁₀ alkoxy group which may be substituted include,methoxy, ethoxy, propoxy, isopropoxy, butoxy, isobutoxy, sec-butoxy, tert-butoxy, trifluoromethyloxy, and benzyloxy. Examples of the C₆-C₂₀ aryloxy group which may be substituted include phenyloxy, o-tolyloxy, m-tolyloxy, p-tolyloxy, o-chlorophenyloxy, m-chlorophenyloxy, p-chlorophenyloxy, o-nitrophenyloxy, m-nitrophenyloxy, and p-nitrophenyloxy. Examples of the C₆-C₂₀ aryl group which may be substituted include phenyl, o-tolyl, m-tolyl, p-tolyl, o-nitrophenyl, m-nitrophenyl, p-nitrophenyl, o-cyanophenyl, m-cyanophenyl, p-cyanophenyl, o-methoxyphenyl, m-methoxyphenyl, p-methoxyphenyl, 3,4-methylenedioxyphenyl, and 2,3-methylenedioxyphenyl. In order to obtain the target compound in high yield, phenyl is preferred.

Both the azodicarboxylic acid ester represented by formula (13) and the phosphine compound represented by formula (14) are preferably used in amounts of 1 to 5 times and more preferably 1 to 3 times the moles of the optically active 2-hydroxymethyl-3-(3,4-methylenedioxyphenyl)propionic acid derivative (8).

The reaction is preferably performed at -78°C to 30°C and more preferably at -78°C to 0°C. The disappearance of the raw materials is generally confirmed by reaction for several hours, but the reaction time is preferably about 2 to 24 hours. The reaction is performed in an organic solvent, for example, benzene, a substituted benzene such as toluene, a chloroalkane such as methylene chloride, an ether, an alkane, or tetrahydrofuran, and preferably tetrahydrofuran, toluene, or methylene chloride.

After the reaction, the insoluble matter is removed by filtration, and the resultant filtrate is concentrated to obtain the target compound. The resulting compound can be supplied to a next step without purification, but the compound may be used after purification by, for example, chromatography or the like.

Next, the cyclization process 2) will be described. The reaction is performed in the coexistence of the sulfonic acid halide (4) and a base. The base allowed to coexist is not particularly limited as long as it can capture the produced acid. Preferred examples of the base include tertiary amines such as triethylamine, diisopropylethylamine, pyridine, dimethylaminopyridine, and lutidine. The amounts of the sulfonic acid halide and base used are preferably 1 to 5 times the moles of the optically active 2-hydroxymethyl-3-(3,4-methylenedioxyphenyl)propionic acid derivative (8), and more preferably 1 to 2 times the moles from an economical viewpoint.

The reaction is performed in a proper organic solvent or without a solvent. As the solvent, benzene, a substituted benzene such as toluene, a chloroalkane such as methylene chloride, an ether, an alkane, or tetrahydrofuran is preferably used, and tetrahydrofuran, toluene, or methylene chloride is more preferably used. Without the solvent, the reaction can be conducted by adding an excess of the base to be allowed to coexist. When the solvent is not used, the base to be added in an excess may be selected from the above-described tertiary amines, but pyridine is preferably used. The reaction temperature is preferably -10°C to 50°C and more preferably -5°C to 30°C. The reaction may be stopped when the raw materials disappear, but the reaction time is preferably about 3 to 10 hours.

In this process, the product may be a mixture containing the optically active β-lactone (2) and a compound containing 3,4-methylenedioxyphenyl as R¹ of the optically active 2-sulfonyloxymethyl-3-phenylpropionic acid (1). For example, the produced compound which contains 3,4-methylenedioxyphenyl as R¹ of the optically active 2-sulfonyloxymethyl-3-phenylpropionic acid (1) can be cyclized by the above-described process in a mixed solvent containing an organic solvent and water to finally obtain the optically active β-lactone (2) as a product.

After the reaction, the reaction solution may be washed with water and then concentrated, and the residue may be supplied to a next step without purification. However, the residue may be used after purification by, for example, chromatography or the like. When a solvent miscible with water, such as tetrahydrofuran, is used as the reaction solvent, the solvent may be removed before water is added, and water and an appropriate organic solvent may be added to perform extraction.

Next, the above-mentioned cyclization process 3) using a condensing agent will be described. Examples of the condensing agent include dicyclohexylcarbodiimide, diisopropylcarbodiimide, and N-ethyl-N'-3-dimethylaminopropylcarbodiimide, hydrochlorides thereof, benzotriazol-1-yl-tris-(dimethylamino)phosphonium hexafluorophosphate, and diphenylphosphoryl azide. The amount of the condensing agent used is preferably 1 to 5 times the moles of the optically active 2-hydroxymethyl-3-(3,4-methylenedioxyphenyl)propionic acid derivative (8), and more preferably 1 to 2 times the moles from an economical viewpoint.

The reaction is performed in a proper organic solvent. As the solvent, benzene, a substituted benzene such as toluene, a chloroalkane such as methylene chloride, an ether, an alkane, or tetrahydrofuran is preferably used, and methylene chloride is more preferably used. The reaction temperature is preferably -10°C to 50°C and more preferably -5°C to 30°C. The reaction may be stopped when the raw materials disappear, but the reaction time is preferably about 5 to 24 hours.

After the reaction, the reaction solution may be washed with water and then concentrated, and the residue may be supplied to a next step without purification. However, the residue may be used after purity is increased by, for example, chromatography or the like. When a solvent miscible with water, such as tetrahydrofuran, is used as the reaction solvent, the solvent may be removed before water is added, and water and an appropriate organic solvent may be added to perform extraction. As the solvent used for extraction, for example, benzene, a substituted benzene such as toluene, a chloroalkane such as methylene chloride, an ether or an alkane is preferably used, and toluene, ethyl acetate, or methylene chloride is more preferably used.

Furthermore, the cyclization process 4) will be described. The halogenated ester is represented by, for example, formula (15): 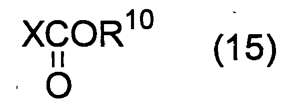 (wherein X represents a halogen atom, and R¹⁰ represents a C₁-C₁₀ alkyl group which may be substituted). As the halogen atom X, an iodine atom, a bromine atom, and a chlorine atom are preferred, and a chlorine atom is more preferred. Examples of the C₁-C₁₀ alkyl group R¹⁰ which may be substituted include methyl, ethyl, propyl, isopropyl, butyl, isobutyl, sec-butyl, and tert-butyl. Among these groups, methyl and ethyl are preferred.

The reaction is performed in coexistence with a base in a proper organic solvent. As the organic solvent, benzene, a substituted benzene such as toluene, a chloroalkane such as methylene chloride, an ether, an alkane, or tetrahydrofuran is preferably used, and tetrahydrofuran or methylene chloride is more preferably used. The base allowed to coexist is not particularly limited as long as it can capture the produced acid. Preferred examples of the base include tertiary amines such as triethylamine, diisopropylethylamine, pyridine, dimethylaminopyridine, and lutidine. The amounts of the halogenated formic acid ester and base used are preferably 1 to 5 times the moles of the optically active 2-hydroxymethyl-3-(3,4-methylenedioxyphenyl)propionic acid derivative (8), and more preferably 1 to 2 times the moles from an economical viewpoint.

The reaction temperature is preferably in a range of -20°C to 30°C and more preferably in a range of -10°C to 10°C. The reaction very rapidly proceeds, and the disappearance of the raw materials is confirmed several minutes after. After the reaction, an insoluble substance such as an inorganic salt or the like can be removed by filtration, and the resultant filtrate can be concentrated to obtain the target compound. The compound can be supplied to a next step without purification, but it can be used after purification by, for example, chromatography or the like.

Finally, the cyclization process 5) will be described. Although the chlorinating agent reacted is not particularly limited, thionyl chloride or the like is preferably used. The amount of the chlorinating agent used is preferably 1 to 50 times the moles of the optically active 2-hydroxymethyl-3-(3,4-methylenedioxyphenyl)propionic acid derivative (8), and more preferably 1 to 20 times the moles from an economical viewpoint.

The reaction is performed in a proper organic solvent. As the organic solvent, benzene, a substituted benzene such as toluene, a chloroalkane such as methylene chloride, an ether, an alkane, tetrahydrofuran, dimethylformamide, or dimethylsulfoxide is preferably used, and dimethylformamide, or dimethylsulfoxide is more preferably used. The reaction temperature is preferably 5°C to 60°C and more preferably in a range of 5°C to 40°C. The reaction may be stopped when the yield is maximized, but the reaction time is preferably about 5 to 120 hours. After the reaction, the reaction solution can be concentrated, and the residue can be supplied to a next step without purification. However, the residue may be used after purity is increased by, for example, chromatography or the like.

The present inventors found a novel compound containing a group other than an unsubstituted phenyl group as R¹ in formula (2), i.e., an optically active β-lactone derivative represented by formula (10): 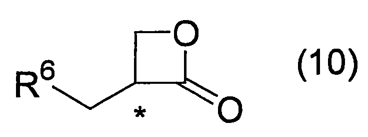 (wherein * represents the same as the above, and R⁶ represents a substituted phenyl group), the novel compound having usefulness for synthesis of optically active 2-thiomethyl-3-phenylpropionic acid.

Next, description will be made of a process of reacting the resultant optically active β-lactone derivative (2) with a sulfur compound represented by formula (6):

R⁴SR⁵ (6)

to produce an optically active 2-thiomethyl-3-phenylpropionic acid derivative represented by formula (7) :

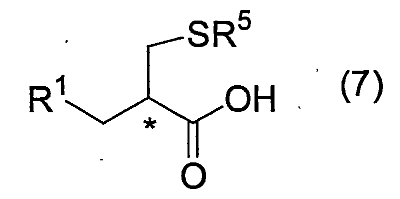

In formula (6), R⁴ represents a hydrogen atom or an alkali metal atom, for example, lithium, sodium, potassium, or the like. In order to obtain the target compound in high yield, a',hydrogen atom or a potassium atom is preferred.

In formulae (6) and (7), R⁵ represents a C₁-C₁₀ alkyl group which may be substituted, a C₆-C₂₀ aryl group which may be substituted, a C₂-C₂₀ acyl group which may be substituted, or a C₇-C₂₀ aroyl group which may be substituted. Examples of the C₁-C₁₀ alkyl group which may be substituted include methyl, ethyl, propyl, isopropyl, butyl, isobutyl, sec-butyl, tert-butyl, trifluoromethyl, and benzyl. Examples of the C₆-C₂₀ aryl group which may be substituted include phenyl, o-tolyl, m-tolyl, p-tolyl, o-chlorophenyl, m-chlorophenyl, p-chlorophenyl, o-methoxyphenyl, m-methoxyphenyl, and p-methoxyphenyl. Examples of the C₁-C₂₀ acyl group which may be substituted include acetyl, ethyryl, propyryl, butyryl, isobutyryl, and trifluoroacetyl. Examples of the C₇-C₂₀ aroyl group which may be substituted include benzoyl, o-toluyl, m-toluyl, p-toluyl, o-anisoyl, m-anisoyl, and p-anisoyl. In order to promote the reaction in high yield, acetyl is preferred.

The amount of the sulfur compound (6) used is preferably 1 to 5 equivalents and more preferably 1 to 2 equivalents relative to the optically active β-lactone (2).

The reaction can be performed in an organic solvent or a two-phase system including an organic solvent and water. When the organic solvent is used alone, dimethylsulfoxide, dimethylformamide, tetrahydrofuran, or acetonitrile is preferably used. These solvents can be used alone or as a mixed solvent of two or more. When the two-phase system including the organic solvent and water is used, ethyl acetate, toluene, acetonitrile, benzene, xylene, methylene chloride, chloroform, or the like is preferably used as the organic solvent. In particular, ethyl acetate or t'oluene is preferably used from the viewpoint of reaction yield and environmental consideration. The quantitative ratio between water and the organic solvent is not particularly limited, but the amount of the organic solvent is preferably 1 to 10 times and more preferably 1 to 5 times the volume of the water.

The reaction temperature is preferably in a range of 0°C to a reflux temperature and more preferably in a range of 0°C to 60°C. Since the disappearance of the raw materials is confirmed by reaction for about 1 to 5 hours, the reaction may be stopped at the disappearance. Post-treatment is performed by distilling off the organic solvent under reduced pressure, adjusting pH of the residue to acid side by adding an acid, and then performing extraction with an organic solvent. As the acid, hydrochloric acid, sulfuric acid, nitric acid, or the like is preferably used. The pH is preferably adjusted in a range of 1 to 7 and more preferably in a range of 1 to 4. Although any of ordinary organic solvents can be used without limitation, toluene, ethyl acetate, methylene chloride, and the like are preferred, and toluene and ethyl acetate are particularly preferred.

The reaction with the sulfur compound containing a hydrogen atom as R⁷ in formula (6) may hardly proceed under the above-described reaction conditions. In this case, an alkali or an amine may be preferably allowed to coexist.

As the alkali, an alkali metal hydroxide, an alkali metal hydrogen carbonate, an alkali metal carbonate, or the like can be used. Preferred examples of the alkali metal hydroxide include sodium hydroxide, lithium hydroxide, and potassium hydroxide. Preferred examples of the alkali metal hydrogen carbonate include sodium hydrogen carbonate, potassium hydrogen carbonate, and lithium hydrogen carbonate. Preferred,examples of the alkali metal carbonate include sodium carbonate, potassium carbonate, and lithium carbonate. When the reaction is performed in coexistence with the alkali, the reaction is performed in a mixed solvent of an organic solvent and water. As the organic solvent, ethyl acetate, toluene, acetonitrile, benzene, xylene, methylene chloride, chloroform, or the like is preferably used, and ethyl acetate or toluene is particularly preferably used from the viewpoint of reaction yield and environmental consideration. The quantitative ratio between the water and the organic solvent is not particularly limited, but the amount of the organic solvent is preferably 1 to 10 times and more preferably 1 to 5 times the volume of the water.

As the amine, triethylamine, diisopropylethylamine, pyridine, dimethylaminopyridine, lutidine, or the like is preferably used. The reaction is preferably performed in an appropriate organic solvent, such as dimethylsulfoxide, dimethylformamide, tetrahydrofuran, or acetonitrile. These solvents can be used alone or as a mixed solvent of two or more.

In use of any of the amines or alkalis, the reaction temperature, the reaction time, the post-treatment, etc. can be determined without any change in the above-described process in which the alkali or amine does not coexist.

### Industrial Applicability

According to the present invention, an optically active 2-thiomethyl-3-phenylpropionic acid derivative useful as an intermediate for medicines can be simply and industrially advantageously produced from inexpensive raw materials.

### Best Mode for Carrying Out the Invention

### [Examples]

The present invention will be described in further detail below with reference to examples, but the present invention is not limited to these examples.

### (Reference Example 1) Ethyl (S)-2-hydroxymethyl-3-(3,4-methylenedioxyphenyl)propionate

Recombinant Escherichia coli HB101 (pTSBG1) Accession No. FERM BP-7119 was inoculated in 50 ml of a 2×YT medium (tripeptone 1.6%, yeast extract 1.0%, NaCl 0.5%, pH = 7.0) sterilized in a 500-ml Sakaguchi flask, followed by shaking culture at 37°C for 18 hours. Then, 0.5 g of ethyl 2-formyl-3-(3,4-methylenedioxyphenyl)propionate, 2.5 mg of NADP, and 0.5 g of glucose were added to 50 ml of the resultant culture solution, followed by stirring at 30°C for 24 hours. After the completion of reaction, the reaction solution was subjected to extraction with toluene and concentration to obtain 0.49 g of a brown oily substance. As a result of analysis of the chemical purity and optical purity of the product by GC (column: TC-FFAP 5m×0.25mm I.D. (manufactured by GL Science Co., Ltd.), carrier gas: He = 30 kPa, detection: FID, column temperature: 150°C) and HPLC (column: Chiralcel AS (manufactured by Daicel Chemical Industries, Ltd.), mobile phase: hexane/isopropanol = 98/2, flow rate: 1 mL/min, detection wavelength: 210 nm, column temperature: 40°C, detection time: R isomer 16'.1 minutes, S isomer 18.3 minutes) it was confirmed that the title compound was obtained with a chemical purity of 96.8%, and an optical purity of 43% ee. ¹H NMR (400 Hz, CDCl₃) δ: 6.73-6.56 (3H, m), 5.93 (2H, s), 4.12-4.23 (2H, q), 3.76-3.64 (2H, m), 2.95-2.69 (3H, m), 1.27 (3H, t)

### (Reference Example 2) Ethyl (R)-2-hydroxymethyl-3-(3,4-methylenedioxyphenyl)propionate

Recombinant Escherichia coli HB101(pNTCRG) Accession No. FERM BP-6898 was inoculated in 50 ml of a 2×YT medium (tripeptone 1.6%, yeast extract 1.0%, NaCl 0.5%, pH = 7.0) sterilized in a 500-ml Sakaguchi flask, followed by shaking culture at 37°C for 18 hours. Then, 87 g of ethyl 2-formyl-3-(3,4-methylenedioxyphenyl)propionate, 27.5 mg of NADP, and 89 g of glucose were added to 550 ml of the resultant culture solution, followed by stirring at 30°C for 24 hours. After the completion of reaction, the reaction solution was subjected to extraction with toluene and concentration to obtain 84.1 g of a brown oily substance. As a result of analysis of the chemical purity and optical purity of the product by the same method as in Reference Example 2, it was confirmed that the title compound was obtained with a chemical purity of 96.5%, and an optical purity of 96.4% ee.

### (Example 1) Ethyl (S)-2-methanesulfonyloxymethyl-3-(3,4-methylenedioxyphenyl)propionate

Ethyl (S)-2-hydroxymethyl-3-(3,4-methylenedioxyphenyl)propionate (70.62 g, 279.94 mmol) which was prepared, for example, as in Reference Example 1, and triethylamine (58.53 mL, 419.91 mmol) were dissolved in toluene (630 mL), and the air in a reactor was replaced with nitrogen. The resultant solution was cooled to an inner temperature of 0°C in an ice bath. Then, methanesulfonyl chloride (32.5 mL, 419.91 mmol) was slowly added dropwise to the solution over about 1.5 hours with the inner temperature kept at 10°C or less. After the completion of the addition, the ice bath was removed, and stirring was further continued for 2 hours. Then, a portion of the reaction solution was extracted and analyzed by HPLC (column: Lichrosphere, mobile phase: aqueous phosphoric acid-potassium dihydrogen phosphate solution/acetonitrile = 1/1, flow rate: 1 mL/min, detection wavelength: 210 nm, column temperature: 30°C). As a result, it was confirmed that the raw material disappeared. The reaction solution was washed with water (400 mL×2). For caution's sake, the washings were subjected to extraction with toluene (500 mL×1), and the toluene solution was added to the washed reaction solution. Then, the solvent was distilled off under reduced pressure to obtain the title compound as red oil. Analysis of the oil by ¹H NMR and HPLC confirmed that the title compound was obtained (92.04 g, purity 96.18 wt%, yield 95.70%).
¹H NMR (400 Hz, CDCl₃) δ: 6.71-6.58 (3H, m), 5.90 (2H, s), 4.33-4.26 (2H, m), 4.13 (2H, m), 3.00-2.74 (4H, m), 1.21 (3H, t)

### (Example 2) Ethyl (R)-2-methanesulfonyloxymethyl-3-(3,4 methylenedioxyphenyl)propionate

The title compound was obtained (102.09 g, purity 92.50 wt%, yield 96.0%) by the same procedure as in Example 1 using ethyl (R)-2-hydroxymethyl-3-(3,4-methylenedioxyphenyl)propionate (75.08 g, 297.62 mmol) which was prepared, for example, was in Reference Example 2. ¹H NMR (400 Hz, CDCl₃) δ: 6.71-6.58 (3H, m), 5.90 (2H, s), 4.33-4.26 (2H, m), 4.13 (2H, m), 3.00-2.74 (4H, m), 1.21 (3H, t)

### (Example 3) (S)-2-Methanesulfonyloxymethyl-3-(3,4-methylenedioxyphenyl)propionic acid

Ethyl (S)-2-methanesulfonyloxymethyl-3-(3,4-methylenedioxyphenyl)propionate (1.92 g, 5.82 mmol) and a 10 wt% aqueous sulfuric acid solution (2.85 g, 2.91 mmol) were added to acetic acid (10 g), and the resultant mixture was stirred at 90°C. Twenty hours after, the mixture was allowed to cool to room temperature, and sodium acetate (477.41 mg, 5.82 mmol) was added. Then, acetic acid was distilled off under reduced pressure. When the total amount was decreased to about 1/3, ethyl acetate (50 mL) was added to the residue, and the resultant mixture was washed three times with water (20 mL). The washings were subjected to extraction with ethyl acetate (30 mL), and the extraction solution was mixed with the ethyl acetate layer previously obtained. The resultant mixture was dried over anhydrous magnesium sulfate, and then the solvent was distilled off under reduced pressure to obtain red oil. Analysis of the oil by ¹H NMR and HPLC (column: COSMOSIL 5C18-AR (Nacalai Inc.), mobile phase: aqueous phosphoric acid-potassium dihydrogen phosphate solution (pH=2)/acetonitrile = 7/3, flow rate: 1 mL/min, detection wavelength: 210 nm, column temperature: 40°C) confirmed that the title compound was obtained (1.99 g, purity 67.80 wt%, yield 76.60%).
¹H NMR (400 Hz, CDCl₃) δ: 9.50 (1H, br), 6.75-6.63 (3H, m), 5.93 (2H, s), 3.07-2.90 (5H, m), 2.85-2.81 (1H, m)

### (Example 4) (S)-2-Methanesulfonyloxymethyl-3-(3,4-methylenedioxyphenyl)propionic acid

Ethyl (S)-2-methanesulfonyloxymethyl-3-(3,4-methylenedioxyphenyl)propionate (250 mg, 740.9 µmol) and a 10 wt% aqueous p-toluenesulfonic acid solution (720.0 mg, 378.4 µmol) were added to acetic acid (2.5 g), and the resultant mixture was stirred at 70°C. Sixty-five hours after, the temperature was increased to 90°C, and the reaction was further performed for 22 hours. The reaction solution was allowed to cool to room temperature and then analyzed by HPLC (under the same analytical conditions as in Example 3). As a result, it was confirmed that the title compound was obtained in a yield of 80.0%.
¹H NMR (400 Hz, CDCl₃) δ: 9.50 (1H, br), 6.75-6.63 (3H, m), 5.93 (2H, s), 3.07-2.90 (5H, m), 2.85-2.81 (1H, m)

### (Example 5) (S)-2-Methanesulfonyloxymethyl-3-(3,4-methylenedioxyphenyl)propionic acid

Ethyl (S)-2-methanesulfonyloxymethyl-3-(3,4-methylenedioxyphenyl)propionate (250 mg, 740.9 µmol) and a 10 wt% aqueous sulfuric acid solution (181.5 mg, 185.23 µmol) were added to acetic acid (2.5 g), and the resultant mixture was stirred at 90°C for 49 hours. Then, the reaction solution was allowed to cool to room temperature and analyzed by HPLC (under the same analytical conditions as in Example 3). As a result, it was confirmed that the title compound was obtained in a yield of 58.0%.
¹H NMR (400 Hz, CDCl₃) δ: 9.50 (1H, br), 6.75-6.63 (3H, m), 5.93 (2H, s), 3.07-2.90 (5H, m), 2.85-2.81 (1H, m)

### (Example 6) (S)-2-Methanesulfonyloxymethyl-3-(3,4-methylenedioxyphenyl)propionic acid

Ethyl (S)-2-methanesulfonyloxymethyl-3-(3,4-methylenedioxyphenyl)propionate (250 mg, 740.9 µmol) and a 10 wt% aqueous sulfuric acid solution (90.8 mg, 92.62 µmol) were added to acetic acid (2.5 g), and the resultant mixture was stirred at 90 °C for 49 hours. Then, the reaction solution was allowed to cool to room temperature and analyzed by HPLC (under the same analytical conditions as in Example 3). As a result, it was confirmed that the title compound was obtained in a yield of 50.0%.
¹H NMR (400 Hz, CDCl₃) δ: 9.50 (1H, br), 6.75-6.63 (3H, m), 5.93 (2H, s), 3.07-2.90 (5H, m), 2.85-2.81 (1H, m)

### (Example 7) (R)-2-Methanesulfonyloxymethyl-3-(3,4-methylenedioxyphenyl)propionic acid

The same procedure as in Example 3 was carried out using ethyl (R)-2-methanesulfonyloxymethyl-3-(3,4-methylenedioxyphenyl)propionate (961.8 mg, 2.91 mmol). HPLC analysis (under the same analytical conditions as in Example 3) of the reaction solution confirmed that the title compound was obtained in a yield of 83%.
¹H NMR (400 Hz, CDCl₃) δ: 9.50 (1H, br), 6.75-6.63 (3H, m), 5.93 (2H, s), 3.07-2.90 (5H, m), 2.85-2.81 (1H, m)

### (Example 8) (S)-3-(3,4-Methylenedioxybenzyl)-2-oxetanone

(S)-2-Methanesulfonyloxymethyl-3-(3,4-methylenedioxyphenyl)propionic acid (3.0 g, 9.92 mmol) was dissolved in ethyl acetate (27 g), and a 30% aqueous NaOH solution (1.72 g) was added to the resultant solution to adjust the pH to 8.14. After stirring at room temperature for 22 hours, the aqueous layer was removed with a separatory funnel, and the organic layer was washed with water. The water washing was stopped when the pH of the water was close to 6. Then, the resultant organic layer was concentrated under reduced pressure to obtain red oil. As a result of analysis of the oil by ¹H NMR and HPLC (column: COSMOSIL 5C18-AR (Nacalai Inc.), mobile phase: aqueous phosphoric acid-potassium dihydrogen phosphate solution (pH=2)/acetonitrile = 6/4, flow rate: 1 mL/min, detection wavelength: 210 nm, column temperature: 40°C), it was confirmed that the title compound was obtained (1.92 g, yield 93.8%).
¹H NMR (400 Hz, CDCl₃) δ: 6.78-6.57 (3H, m), 5.90 (2H, s), 4.35 (1H, m), 4.05-3.90 (2H, m), 3.10-2.96 (2H, m)

### (Example 9) (S)-3-(3,4-Methylenedioxybenzyl)-2-oxetanone

(S)-2-Methanesulfonyloxymethyl-3-(3,4-methylenedioxyphenyl)propionic acid (3.0 g, 9.92 mmol) was dissolved in ethyl acetate (27 g), and a 1M aqueous NaOH solution (4.04 g) was added to the resultant solution to adjust the pH to 5.99. After stirring at 40°C for 8 hours, the reaction solution was analyzed by HPLC (under the same analytical conditions as in Example 8), it was confirmed that the title compound was obtained in a yield of 40.1%. ¹H NMR (400 Hz, CDCl₃) δ: 6.78-6.57 (3H, m), 5.90 (2H, s), 4.35 (1H, m), 4.05-3.90 (2H, m), 3.10-2.96 (2H, m)

### (Example 10) (R)-3-(3,4-Methylenedioxybenzyl)-2-oxetanone

(R)-2-Mesyloxymethyl-3-(3,4-methylenedioxyphenyl)propionic acid (267.13 mg, 883.67 µmol) was cyclized by the same procedure as in Example 8. Analysis of the reaction solution by HPLC (under the same analytical conditions as in Example 8) confirmed that the title compound was obtained in a yield of 89%.
¹H NMR (400 Hz, CDCl₃) δ: 6.78-6.57 (3H, m), 5.90 (2H, s), 4.35 (1H, m), 4.05-3.90 (2H, m), 3.10-2.96 (2H, m)

### (Example 11) (S)-2-Hydroxymethyl-3-(3,4-methylenedioxyphenyl)propionic acid

Ethyl (S)-2-hydroxymethyl-3-(3,4-methylenedioxyphenyl)-propionate (252.26 mg, 1.0 mmol), which was prepared, for example, as in Reference Example 1, was dissolved in isopropyl alcohol (3 mL), and the resultant solution was cooled to 0°C. Then, an aqueous solution (1 mL) of sodium hydroxide (80 mg) was added to the solution, and the mixture was stirred at 0°C for 13 hours. Then, water (10 mL) was added to the mixture, and the resultant solution was washed with ethyl acetate (10 mL). The aqueous layer was adjusted to a pH of 1 to 3 with conc. hydrochloric acid, followed by extraction with ethyl acetate (10 mL×2). The organic layer was dried over anhydrous magnesium sulfate, and the solvent was distilled off under reduced pressure to obtain a white solid. Analysis of the white solid by ¹H NMR and HPLC (under the same analytical conditions as in Example 3) confirmed that the title compound was obtained (231.0 mg, yield 98.80%).
¹H NMR (400 Hz, CDCl₃) δ: 6.75-6.58 (3H, m), 5.90 (2H, s), 3.80-3.61 (2H, m), 3.00-2.73 (3H, m)

### (Example 12) (R)-2-Hydroxymethyl-3-(3,4-methylenedioxyphenyl)propionic acid

The tile compound was obtained (224.0 mg, yield 95.80%) by the same procedure as in Example 11 using ethyl (R)-2-hydroxymethyl-3-(3,4-methylenedioxyphenyl)propionate (252.26 mg, 1.0 mmol), which was prepared, for example, as in Reference Example 2.
¹H NMR (400 Hz, CDCl₃) δ: 6.75-6.58 (3H, m), 5.90 (2H, s), 3.80-3.61 (2H, m), 3.00-2.73 (3H, m)

### (Example 13) (S)-3-(3,4-Methylenedioxybenzyl)-2-oxetanone

(S)-2-Hydroxymethyl-3-(3,4-methylenedioxyphenyl)propionic acid (250 mg, 1.04 mmol) was dissolved in tetrahydrofuran (2.5 mL) in a nitrogen atmosphere, and the resultant solution was cooled to -5°C. Then, triethylamine (144.96 µL, 1.04 mmol) and ethyl chloroformate (99.35 µL, 1.04 mmol) were added to the solution, followed by stirring at -5°C. Fifteen minutes after, the temperature was increased to 0°C, and the reaction was further performed for 15 minutes. Analysis of the reaction solution by HPLC (under the same analytical conditions as in Example 8) confirmed that the title compound was obtained in a yield 68.2%.
¹H NMR (400 Hz, CDCl₃) δ: 6.78-6.57 (3H, m), 5.90 (2H, s), 4.35 (1H, m), 4.05-3.90 (2H, m), 3.10-2.96 (2H, m)

### (Example 14) (S)-3-(3,4-Methylenedioxybenzyl)-2-oxetanone

Triphenylphosphine (262.3 mg, 1.0 mmol) was dissolved in tetrahydrofuran (4 mL) in a nitrogen atmosphere, and the resultant solution was cooled to -78°C. Then, a tetrahydrofuran (4 mL) solution of isopropyl azodicarboxylate (202.2, mg, 1.0 mmol) was added dropwise to the solution over about 10 minutes. After the completion of the addition, the mixture was further stirred for 10 minutes, and a tetrahydrofuran (4 mL) solution of (S)-2-hydroxymethyl-3-(3,4-methylenedioxyphenyl)propionic acid (224.0 mg, 1.0 mmol) was added dropwise to the mixture over about 10 minutes. After the completion of the addition, stirring was performed for 20 minutes and then further performed for 3 hours after the temperature was returned to room temperature. Analysis of the reaction solution by HPLC (under the same analytical conditions as in Example 8) confirmed that the title compound was obtained in a yield 72.2%.
¹H NMR (400 Hz, CDCl₃) δ: 6.78-6.57 (3H, m), 5.90 (2H, s), 4.35 (1H, m), 4.05-3.90 (2H, m), 3.10-2.96 (2H, m)

### (Example 15) (S)-3-(3,4-Methylenedioxybenzyl)-2-oxetanone

(S)-2-Hydroxymethyl-3-(3,4-methylenedioxyphenyl) propionic acid (116.4 mg, 1.0 mmol) wad dissolved in pyridine (1 mL) in a nitrogen atmosphere, and the resultant solution was cooled to -20°C. Then, methanesulfonyl chloride (0.12 ml) was slowly added dropwise to the solution. After the completion of the addition, the mixture was' stirred at the same temperature for 1 hour. Analysis of the reaction solution by HPLC (under the same analytical conditions as in Example 8) confirmed that the title compound was obtained in a yield 4.9%, and (S)-2-methanesulfonyloxymethyl-3-(3,4-methylenedioxyphenyl)propionic acid was produced in a yield of 23.7%.

### (Example 16) (S)-2-Acetylthiomethyl-3-(3,4-methylenedioxyphenyl)propionic acid

(S)-3-(3,4-Methylenedioxybenzyl)-2-oxetanone (0.80 g, 3.88 mmol) was added to a mixture of toluene (12 mL) and water (4 mL) in a nitrogen atmosphere, and potassium thioacetate (665.00 mg, 5.82 mmol) was added to the resultant mixture, followed by heating to 40°C. Two hours after, the mixture was cooled to 5°C and then adjusted to pH 1 by adding 97% sulfuric acid. The aqueous layer was removed, and the residual organic layer was washed with water two times. Then, the organic layer was concentrated to obtain yellow oil. Analysis of the yellow oil by ¹H NMR and HPLC (under the same analytical conditions as in Example 8) confirmed that the title compound was obtained (937.00 mg, yield 85.5%).
¹H NMR (400 Hz, CDCl₃) δ: 6.78-6.57 (3H, m), 5.90 (2H, s), 3.22-2.79 (5H, m), 2.32 (3H, s)

### (Example 17) (S)-2-Acetylthiomethyl-3-(3,4-methylenedioxyphenyl)propionic acid

(S)-3-(3,4-Methylenedioxybenzyl)-2-oxetanone (0.80 g, 3.88 mmol) was added to a mixture of ethyl acetate (12 mL) and water (4 mL) in a nitrogen atmosphere, and potassium thioacetate (665.00 mg, 5.82 mmol) was added to the resultant mixture, followed by heating to 40°C. Two hours after, the mixture was cooled to 5°C and then adjusted to pH 1 by adding 97% sulfuric acid. The aqueous layer was removed, and the residual organic layer was washed with water two times. Then, the organic layer was concentrated to obtain yellow oil. Analysis of the yellow oil by ¹H NMR and HPLC (column: COSMOSIL 5C18-AR (Nacalai Inc.), mobile phase: aqueous phosphoric acid-potassium dihydrogen phosphate solution (pH=2)/acetonitrile = 7/3, flow rate: 1 mL/min, detection wavelength: 210 nm, column temperature: 40°C) confirmed that the title compound was obtained (927.00 mg, yield 84.6%). ¹H NMR (400 Hz, CDCl₃) δ: 6.78-6.57 (3H, m), 5.90 (2H, s), 3.22-2.79 (5H, m), 2.32 (3H, s)

### (Example 18) (R)-2-Acetylthiomethyl-3-(3,4-methylenedioxyphenyl)propionic acid

(R)-3-(3,4-Methylenedioxybenzyl)-2-oxetanone (240 mg, 1.16 mmol) was reacted by the same procedure as in Example 16. Analysis of the reaction solution byHPLC (under the same analytical conditions as in Example 8) confirmed that the title compound was obtained in a yield of 81.5%.
¹H NMR (400 Hz, CDCl₃) δ: 6.78-6.57 (3H, m), 5.90 (2H, s), 3.22-2.79 (5H, m), 2.32 (3H, s)

## Claims

1. A process for producing an optically active β-lactone derivative represented by formula (2): 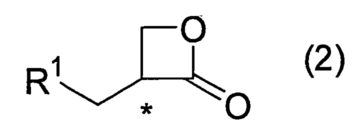 (wherein * represents an asymmetric carbon atom, and R¹ represents a phenyl group which may be substituted), the process comprising cyclizing an optically active 2-sulfonyloxymethyl-3-phenylpropionic acid derivative represented by formula (1): 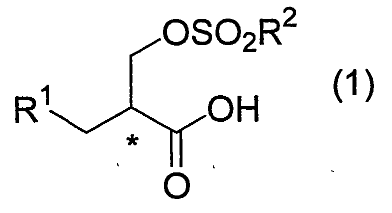 (wherein * and R¹ represent the same as the above, and R² represents a C₁-C₁₀ alkyl group which may be substituted or a C₆-C₂₀ aryl group which may be substituted).

2. The process according to claim 1, wherein cyclization reaction is performed in a mixed solvent containing water and an organic solvent.

3. The process according to claim 2, wherein at least one selected from the group consisting of toluene, benzene, xylene, anisole, ethyl acetate, diethyl ether, methylene chloride, chloroform, and carbon tetrachloride is used as the organic solvent.

4. The process according to any one of claims 1 to 3, wherein the cyclization reaction is performed at a pH of 4 or higher.

5. The process according to any one of claims 1 to 3, wherein the cyclization reaction is performed in a pH range of 4 to 12.

6. The process according to any one of claims 1 to 5, wherein the optically active 2-sulfonyloxymethyl-3-phenylpropionic acid derivative represented by formula (1) is obtained by hydrolyzing an optically active 2-sulfonyloxymethyl-3-phenylpropionic acid ester derivative represented by formula (5) : 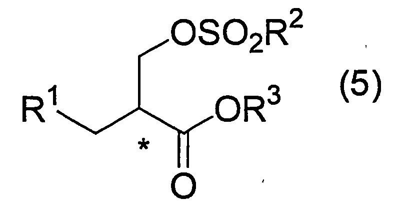 (wherein *, R¹, and R² represent the same as the above, and R³ represents a C₁-C₁₀ alkyl group which may be substituted or a C₆-C₂₀ aryl group which may be substituted by a C₆-C₂₀ group), the derivative represented formula (5) being produced by reacting an optically active 2-hydroxymethyl-3-phenylpropionic acid ester derivative represented by formula (3) : 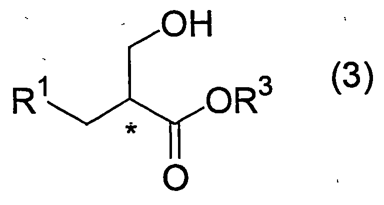 (wherein *, R¹, and R³ represent the same as the above) with a sulfonic acid halide represented by formula (4):
R²SO₂X (4)
(wherein R² represents the same as the above, and X represents a halogen atom).

7. The process according to claim 6, wherein hydrolysis is performed with at least one acid selected from the group consisting of acetic acid, formic acid, hydrochloric acid, sulfuric acid, p-toluenesulfonic acid, methanesulfonic acid, and trifluoromethane sulfonic acid.

8. The process according to claim 6, wherein hydrolysis is performed with sulfuric acid or p-toluenesulfonic acid and acetic acid.

9. The process according to any one of claims 6 to 8, wherein hydrolysis is performed at a temperature in a range of 50°C to a reflux temperature.

10. The process according to any one of claims 6 to 9, wherein R² is methyl, p-tolyl, phenyl, benzyl, or trifluoromethyl.

11. The process according to any one of claims 6 to 10, wherein R³ is methyl, ethyl, or tert-butyl.

12. A process for producing an optically active 2-thiomethyl-3-phenylpropionic acid derivative represented by formula (7): 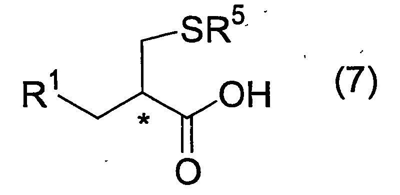 (wherein * represents an asymmetric carbon atom, R¹ represents a phenyl group which may be substituted, and R⁵ represents a C₁-C₁₀ alkyl group which may be substituted, a C₆-C₂₀ aryl group which may be substituted, a C₂-C₂₀ acyl group which may be substituted, or a C₇-C₂₀ aroyl group which may be substituted), the process comprising reacting an optically active β-lactone derivative represented by formula (2): 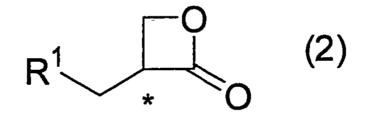 (wherein * and R¹ represent the same as the above) with a sulfur compound represented by formula (7):
R⁴SR⁵ (6)
(wherein R⁴ represents a hydrogen atom or an alkali metal atom, and R⁵ represents the same as the above).

13. The process according to claim 12, wherein the optically active β-lactone derivative represented by formula (2) is produced by the process according to any one of claims 1 to 11.

14. The process according to claim 12 or 13, wherein R⁴ is a hydrogen atom or a potassium atom.

15. The process according to any one of claims 12 to 14, wherein R⁵ is acetyl.

16. The process according to any one of claims 1 to 15, wherein R¹ is any one selected from the group consisting of phenyl, 2,3-methylenedioxyphenyl, 2,3-ethylenedioxyphenyl, 2,3-propylenedioxyphenyl, 3,4-methylenedioxyphenyl, 3,4-ethylenedioxyphenyl, 3,4-propylenedioxyphenyl, o-tolyl, m-tolyl, p-tolyl, 2,3-xylyl, 2,4-xylyl, 2,5-xylyl, 2,6-xylyl, 3,4-xylyl, o-phenoxyphenyl, m-phenoxyphenyl, p-phenoxyphenyl, o-phenylphenyl, m-phenylphenyl, p-phenylphenyl, o-chlorophenyl, m-chlorophenyl, p-chlorophenyl, o-bromophenyl, m-bromophenyl, p-bromophenyl, o-fluorophenyl, m-fluorophenyl, p-fluorophenyl, o-nitrophenyl, m-nitrophenyl, p-nitrophenyl, o-cyanophenyl, m-cyanophenyl, p-cyanophenyl, o-hydroxyphenyl, m-hydroxyphenyl, p-hydroxyphenyl, o-methoxyphenyl, m-methoxyphenyl, p-methoxyphenyl, 2,3-dimethoxyphenyl, 2,4-dimethoxyphenyl, 2,5-dimethoxyphenyl, 2,6-dimethoxyphenyl, 3,4-dimethoxyphenyl, 2,3-difluorophenyl, 2,4-difluorophenyl, 2,5-difluorophenyl, 2,6-difluorophenyl, 3,4-difluorophenyl, 2,3-dihydroxyphenyl, 2,4-dihydroxyphenyl, 2,5-dihydroxyphenyl, 2,6-dihydroxyphenyl, and 3,4-dihydroxyphenyl.

17. The process according to any one of claims 1 to 16, wherein R¹ is phenyl or 3,4-methylenedioxyphenyl.

18. The process according to claims 12, wherein the optically active β-lactone derivative represented by formula (2) is produced by cyclizing an optically active 2-hydroxymethyl-3-(3,4-methylenedioxyphenyl)propionic acid derivative represented by formula (8): 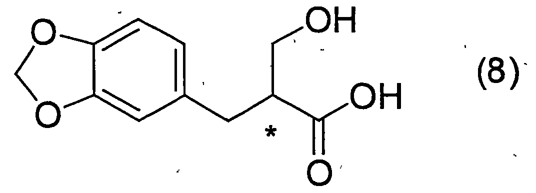 (wherein * represents an asymmetric carbon atom).

19. The process according to claims 18, wherein the optically active 2-hydroxymethyl-3-(3,4-methylenedioxyphenyl)propionic acid derivative represented by formula (8) is produced by hydrolyzing an optically active 2-hydroxymethyl-3-(3,4-methylenedioxyphenyl)propionic acid ester derivative represented by formula (9): 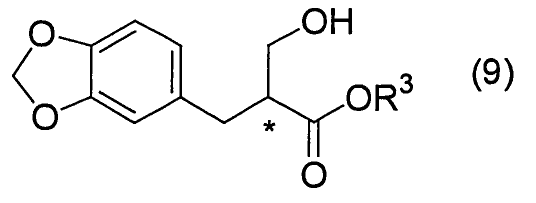 (wherein * represents an asymmetric carbon atom, and R³ represents a C₁-C₁₀ alkyl group which may be substituted, or a C₆-C₂₀ aryl group which may be substituted).

20. The process according to claim 19, wherein R³ is methyl, ethyl, or tert-butyl.

21. The process according to any one of claims 1 to 20, wherein the asymmetric carbon atom has an S absolute configuration.

22. The process according to any one of claims 1 to 20, wherein the asymmetric carbon atom has an R absolute configuration.

23. An optically active β-lactone derivative represented by formula (10): 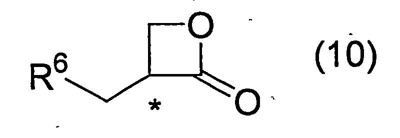 (wherein * represents an asymmetric carbon atom, and R⁶ represents a substituted phenyl group).

24. The compound according to claim 23, wherein R⁶ is any one selected from the group consisting of 2,3-methylenedioxyphenyl, 2,3-ethylenedioxyphenyl, 2,3-propylenedioxyphenyl, 3,4-methylenedioxyphenyl, 3,4-ethylenedioxyphenyl, 3,4-propylenedioxyphenyl, o-tolyl, m-tolyl, p-tolyl, 2,3-xylyl, 2,4-xylyl, 2,5-xylyl, 2,6-xylyl, 3,4-xylyl, o-phenoxyphenyl, m-phenoxyphenyl, p-phenoxyphenyl, o-phenylphenyl, m-phenylphenyl, p-phenylphenyl, o-chlorophenyl, m-chlorophenyl, p-chlorophenyl, o-bromophenyl m-bromophenyl, p-bromophenyl, o-fluorophenyl, m-fluorophenyl, p-fluorophenyl, o-nitrophenyl, m-nitrophenyl, p-nitrophenyl, o-cyanophenyl, m-cyanophenyl, p-cyanophenyl, o-hydroxyphenyl, m-hydroxyphenyl, p-hydroxyphenyl, o-methoxyphenyl, m-methoxyphenyl, p-methoxyphenyl, 2,3-dimethoxyphenyl, 2,4-dimethoxyphehyl, 2,5-dimethoxyphenyl, 2,6-dimethoxyphenyl, 3,4-dimethoxyphenyl, 2,3-difluorophenyl, 2,4-difluorophenyl, 2,5-difluorophenyl, 2,6-difluorophenyl, 3,4-difluorophenyl, 2,3-dihydroxyphenyl, 2,4-dihydroxyphenyl, 2,5-dihydroxyphenyl, 2,6-dihydroxyphenyl, and 3,4-dihydroxyphenyl.

25. The compound according to claim 23, wherein R⁶ is 3,4-methylenedioxyphenyl.

26. An optically active 2-sulfonyloxymethyl-3-phenylpropionic acid ester derivative represented by formula (11) : 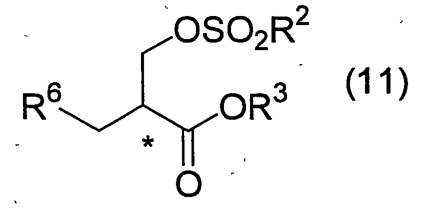 (wherein * represents an asymmetric carbon atom, R⁶ represents a substituted phenyl group, R² represents a C₁-C₁₀ alkyl group which may be substituted or a C₆-C₂₀ aryl group which may be substituted, and R³ represents a C₁-C₁₀ alkyl group which may be substituted or a C₆-C₂₀ aryl group which may be substituted).

27. The compound according to claim 26, wherein R² is methyl, p-tolyl, phenyl, benzyl, or trifluoromethyl.

28. The compound according to claim 26 or 27, wherein R³ is methyl, ethyl, or tert-butyl.

29. The compound according to any one of claims 26 to 28, wherein R⁶ is any one selected from the group consisting of 3,4-methylenedioxyphenyl, 3,4-ethylenedioxyphenyl, 3,4-propylenedioxyphenyl, o-tolyl, m-tolyl, p-tolyl, 2,3-xylyl, 2,4-xylyl, 2,5-xylyl, 2,6-xylyl, 3,4-xylyl, o-chlorophenyl, m-chlorophenyl, p-chlorophenyl, o-bromophenyl, m-bromophenyl, p-bromophenyl, o-fluorophenyl, m-fluorophenyl, p-fluorophenyl, o-nitrophenyl, m-nitrophenyl, p-nitrophenyl, o-cyanophenyl, m-cyanophenyl, p-cyanophenyl, o-hydroxyphenyl, m-hydroxyphenyl, p-hydroxyphenyl, o-methoxyphenyl, m-methoxyphenyl, p-methoxyphenyl, 2,3-dimethoxyphenyl, 2,4-dimethoxyphenyl, 2,5-dimethoxyphenyl, 2,6-dimethoxyphenyl, and 3,4-dimethoxyphenyl.

30. An optically active 2-hydroxymethyl-3-phenylpropionic acid derivative represented by formula (12): 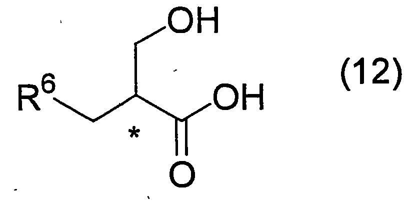 (wherein * represents an asymmetric carbon atom, and R⁶ represents a substituted phenyl group).

31. The compound according to claim 30, wherein R⁶ is any one selected from the group consisting of 2,3-methylenedioxyphenyl, 2,3-ethylenedioxyphenyl, 2,3-propylenedioxyphenyl, 3,4-methylenedioxyphenyl, 3,4-ethylenedioxyphenyl, 3,4-propylenedioxyphenyl, 2,3-xylyl, 2,4-xylyl, 2,5-xylyl, 2,6-xylyl, 3,4-xylyl, o-chlorophenyl, m-chlorophenyl, p-chlorophenyl, o-bromophenyl, m-bromophenyl, p-bromophenyl, o-fluorophenyl, m-fluorophenyl, p-fluorophenyl, o-nitrophenyl, m-nitrophenyl, p-nitrophenyl, o-cyanophenyl, m-cyanophenyl, p-cyanophenyl, o-hydroxyphenyl, m-hydroxyphenyl, p-hydroxyphenyl, o-methoxyphenyl, m-methoxyphenyl, p-methoxyphenyl, 2,3-dimethoxyphenyl, 2,4-dimethoxyphenyl, 2,5-dimethoxyphenyl, 2,6-dimethoxyphenyl, and 3,4-dimethoxyphenyl.

32. The compound according to any one of claims 23 to 31, wherein the asymmetric carbon atom has an S absolute configuration.

33. The compound according to any one of claims 23 to 31, wherein the asymmetric carbon atom has an R absolute configuration.
